**Europäisches Patentamt**

**European Patent Office**

**Office européen des brevets**

(11) Veröffentlichungsnummer: **0 061 723**
**A2**

(12) # EUROPÄISCHE PATENTANMELDUNG

(21) Anmeldenummer: **82102481.7**

(51) Int. Cl.³: **A 61 M 1/00**

(22) Anmeldetag: **25.03.82**

(30) Priorität: **27.03.81 DE 3112148**

(43) Veröffentlichungstag der Anmeldung:
**06.10.82 Patentblatt 82/40**

(84) Benannte Vertragsstaaten:
**AT BE CH DE FR GB IT LI LU NL SE**

(71) Anmelder: **Sterimed Gesellschaft für medizinischen Bedarf mbH**
**Fasanerieweg 15**
**D-6600 Saarbrücken(DE)**

(72) Erfinder: **Kremling, Willy**
**Forststrasse 10**
**D-6601 Kleinblittersdorf(DE)**

(54) **Saugflasche für die Wunddrainage mit einem Druckanzeiger.**

(57) Eine Saugflasche (1) für die Wunddrainage weist als Druckanzeiger einen durchsichtigen, mit einer Skala (9) versehenen Zylinder (6) mit einem Kolben (10) auf. Der eine Zylinderraum ist mit dem Inneren der Saugflasche (1) verbunden. Der andere Zylinderraum ist durch einen Deckel (8) verschlossen und mit einer abgemessenen Gasfüllung versehen. Der Kolben besteht aus einem steifen Kern (11) mit einem Mantel (12) aus Silikonkautschuk. Die Innenwandung des Zylinders ist silikonisiert. Ein solcher Druckanzeiger ist dicht und genau und außerdem billig.

EP 0 061 723 A2

./...

Fig. 1

Saugflasche für die Wunddrainage mit einem Druckanzeiger

Technisches Gebiet

Die Erfindung betrifft eine Saugflasche für die Wunddrainage mit einem Druckanzeiger.

Stand der Technik

Druckanzeiger an solchen Saugflaschen sind bisher in Form elastischer Membranen bekannt, deren mehr oder weniger starke Einwölbung das Ausmaß des Unterdrucks in etwa erkennbar macht, und in Form von elastischen Faltenbälgen, die mit dem Grad ihrer Zusammenziehung den Unterdruck anzeigen bzw. mit ihrer Auslängung das Nachlassen des Unterdrucks.

Die Anzeige des Faltenbalgs ist infolge seiner größeren Formänderung deutlicher als die der Membran. Sie ist jedoch nicht befriedigend genau reproduzierbar, jedenfalls, wenn die Wandung des Faltenbalgs dick genug bemessen wird, um die erforderliche Dichtheit zu gewährleisten. Bei längerer Lagerzeit der unter Unterdruck gesetzten Saugflasche leidet die Reproduzierbarkeit auch unter Ermüdung des Faltenbalgmaterials.

Darstellung der Erfindung

Der Erfindung liegt die Aufgabe zugrunde, eine Saugflasche für die Wunddrainage mit einem Druckanzeiger zu versehen, der genau und dicht ist. Da es sich um ein Wegwerferzeugnis handelt, muß er trotzdem aber einfach und billig sein.

Gemäß der Erfindung ist ein Druckanzeiger aus einem einen Kolben in erkennbarer Stellung enthaltenden Zylinder vorgesehen, dessen einer Zylinderraum mit dem Inneren der Saugflasche verbunden und dessen anderer Zylinderraum verschlossen und mit einer abgemessenen Gasfüllung versehen ist.

Die Gasfüllung dehnt sich beim Evakuieren der Saugflasche unter Verschiebung des Kolbens im Maße der Druckverminderung in der Saugflasche aus und wird beim Gebrauch der Saugflasche durch den darin ansteigenden Druck
wieder im Maße der Druckerhöhung zusammengedrückt, wiederum unter Verschiebung des Kolbens, der damit den in der Saugflasche vorhandenen Druck
bzw. den noch verfügbaren Unterdruck anzeigt.

Die Wanddicke des Zylinders und seines Verschlusses ist von der Anzeigefunktion völlig unabhängig und kann daher beliebig und ausreichend bemessen werden. Die Anzeige beruht nicht auf elastischer Materialverformung,
sondern auf der Druckabhängigkeit des Volumens der abgemessenen Gasfüllung. Die Kolbendichtung steht auch nicht unter der Belastung eines
Druckunterschieds; der Kolben gibt vielmehr mit seiner Stellung jedem
Druckunterschied nach und läßt ihn sich ausgleichen. Die Berührungskraft
zwischen Kolben und Zylinderwandung kann unter diesen Umständen von vornherein gering sein und dementsprechend dauerhaft.

Nach einer besonders vorteilhaften Weiterbildung der Erfindung besteht
der Zylinder aus einem durchscheinenden oder durchsichtigen Material,
so daß der Kolben selbst sichtbar ist und seine Stellung an einer an dem
Zylinder angeordneten Skala unmittelbar abgelesen werden kann.

Weitere, gesonderte Anzeigeeinrichtungen, etwa mit einem über eine Kolbenstange bewegten Zeiger, sind damit unnötig.

Der Kolben weist vorzugsweise einen steifen Kern und am Umfang einen gerillten Mantel aus Silikonkautschuk auf. Der Kern gibt dem Kolben die
Form und Formbeständigkeit und gewährleistet, daß der Kolben auch bei
seiner Verschiebung genauen Sitz hält und sich nicht verkantet. Der Mantel sichert Dichtheit und Gleitfähigkeit.

Zur Verbesserung der Gleitfähigkeit kann außerdem die Innenwandung des
Zylinders silikonisiert werden.

Der Zylinder sitzt zweckmäßigerweise unmittelbar auf einem an der Saugflasche angeformten Stutzen.

Die Zeichnung gibt ein Ausführungsbeispiel der Erfindung wieder.

Fig. 1 zeigt eine Saugflasche teils im Längsschnitt, teils in Ansicht.
Fig. 2 und
Fig. 3 zeigen ein Teil der Saugflasche in anderen Stellungen.

Eine Saugflasche 1 weist in bekannter Gestaltung einen Anschlußstutzen 2 für einen mittels einer Schlauchklemme 3 dicht verschließbaren, kurzen Schlauch 4 aus einem weichen Material und einen an diesen angeschlossenen Saugschlauch 5 auf.

Nach der vorliegenden Erfindung ist die Saugflasche ferner mit einem Zylinder 6 aus einem weitgehend durchsichtigen Kunststoff versehen, der mit seinem einen Ende auf einem weiteren Stutzen 7 der Saugflasche 1 sitzt und an seinem anderen Ende durch einen Deckel 8 verschlossen ist; beide Verbindungen sind dicht verklebt oder durch ein Lösungsmittel verschweißt o. dgl.. Die Innenwandung des Zylinders 6 ist silikonisiert, auf der Aussenseite ist eine Skala 9 angebracht. In dem Zylinder 6 befindet sich ein Kolben 10. Er weist auf einem steifen Kern 11 einen Mantel 12 aus Silikonkautschuk auf, an dessen Umfang Rillen 13 eingeformt sind.

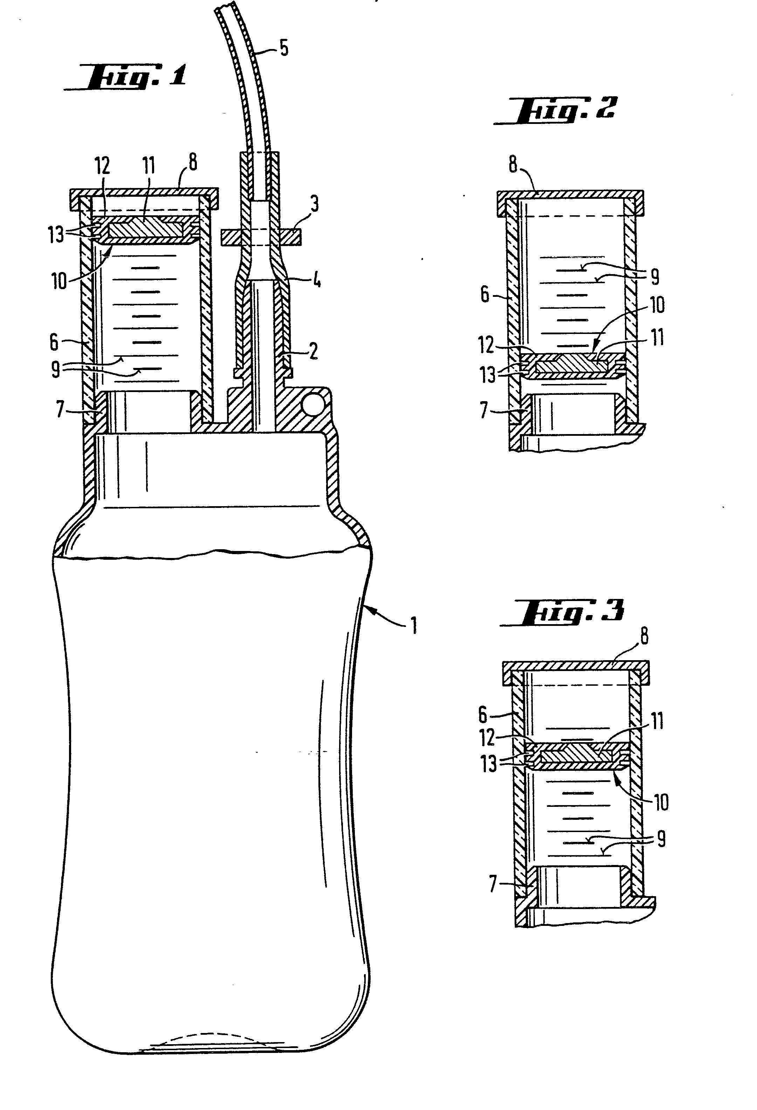

Fig. 1 stellt den Zustand des Kolbens 10 in dem Zylinder 6 vor dem Evakuieren der Saugflasche 1 sowie nach ihrem Gebrauch, das heißt nach vollständiger Wiederherstellung des Atmosphärendrucks in der Saugflasche dar. In diese Stellung ist der Kolben 10 bei der Fertigung vor dem Aufsetzen des Deckels 8 gebracht worden, vorzugsweise mittels einer am oberen Rand des Zylinders 6 sich aufsetzenden Lehre. In den solchermaßen genau abgemessenen Raum oberhalb des Kolbens 10 ist beim Aufsetzen des Deckels 8 Luft unter Atmosphärendruck eingeschlossen worden, also eine bestimmte Menge. Die Ausdehnung dieser Menge beim Evakuieren der Flasche, die durch Verschiebung des Kolbens 10 sichtbar wird, bildet ein reproduzierbares Maß für den dann in der Flasche, durch Auspumpen, eingerichteten Unterdruck. Die Skala 9 macht den Druck ablesbar.

Die Länge des Zylinders 6 unterhalb der Ausgangsstellung des Kolbens 10 ist so bemessen, daß der Kolben bei dem vorgesehenen Enddruck in der Flasche, z.B. 5,33 kPa (40 Torr), eine Stellung nahe dem unteren Ende des Zylinders einnimmt, wie in Fig 2 dargestellt.

**0061723**

Fig. 3 zeigt schließlich den Kolben 10 in seiner eigentlichen Funktion, nämlich in einer beim Gebrauch der Flasche eingenommenen Zwischenstellung, die den noch zur Verfügung stehenden Unterdruck anzeigt.

Soll die Flasche, z.B. für die Neurochirurgie, nur mit einem schwachen Unterdruck versehen sein, so wird man den Zylinderraum über dem Kolben 10 im Verhältnis zu dem Zylinderraum unter dem Kolben 10 so vergrößern, daß sich wiederum der Verschiebeweg des Kolbens im wesentlichen über die übrige Länge des Zylinders erstreckt. Für besonders geringe Unterdrücke könnte man auch den Zylinderdurchmesser verkleinern und den oberen Zylinderraum mit einem dort angesetzten Behälter erweitern.

Patentansprüche

1. Saugflasche für die Wunddrainage mit einem Druckanzeiger, dadurch gekennzeichnet, daß der Druckanzeiger aus einem einen Kolben (10) in erkennbarer Stellung enthaltenden Zylinder (6) besteht, dessen einer Zylinderraum mit dem Inneren der Saugflasche verbunden und dessen anderer Zylinderraum verschlossen (8) und mit einer abgemessenen Gasfüllung versehen ist.

2. Saugflasche nach Anspruch 1, dadurch gekennzeichnet, daß der Zylinder (6) unmittelbar auf einem an der Saugflasche (1) angeformten Stutzen (7) sitzt.

3. Saugflasche nach Anspruch 1 oder 2, dadurch gekennzeichnet, daß der Zylinder (6) aus einem durchscheinenden oder durchsichtigen Material besteht und der Kolben (10) unmittelbar sichtbar ist.

4. Saugflasche nach Anspruch 3, dadurch gekennzeichnet, daß der Zylinder (6) mit einer Skala (9) versehen ist.

5. Saugflasche nach einem der Ansprüche 1 bis 4, dadurch gekennzeichnet, daß der Kolben (10) einen steifen Kern (11) und am Umfang einen, vorzugsweise gerillten (13), Mantel (12) aus Silikonkautschuk aufweist.

6. Saugflasche nach einem der Ansprüche 1 bis 5, dadurch gekennzeichnet, daß die Innenwandung des Zylinders (6) silikonisiert ist.

**Fig. 1**

**Fig. 2**

**Fig. 3**